(19) Europäisches Patentamt
European Patent Office
Office européen des brevets

(11) Veröffentlichungsnummer: **0 013 880**
**B1**

(12) # EUROPÄISCHE PATENTSCHRIFT

(45) Veröffentlichungstag der Patentschrift: **30.12.81**

(51) Int. Cl.³: **C 07 D 251/34,** C 08 G 18/79

(21) Anmeldenummer: **80100026.6**

(22) Anmeldetag: **04.01.80**

(54) **Neue Isocyanato-isocyanurate, ein Verfahren zu ihrer Herstellung sowie ihre Verwendung als Isocyanatkomponente in Polyurethan-Lacken.**

(30) Priorität: **16.01.79 DE 2901479**

(43) Veröffentlichungstag der Anmeldung: **06.08.80 Patentblatt 80/16**

(45) Bekanntmachung des Hinweises auf die Patenterteilung: **30.12.81 Patentblatt 81/52**

(84) Benannte Vertragsstaaten: **BE DE FR GB IT NL**

(56) Entgegenhaltungen: **DE-A-23 25 826**

(73) Patentinhaber: **BAYER AG, Zentralbereich Patente, Marken und Lizenzen, D-5090 Leverkusen 1, Bayerwerk (DE)**

(72) Erfinder: **Breidenbach, Peter, Dr., Maarweg 33, D-5000 Koeln 41 (DE)**
Erfinder: **Pedain, Josef, Dr., Haferkamp 6, D-5000 Koeln 80 (DE)**

EP 0 013 880 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Isocyanato-isocyanurate, ein Verfahren zu ihrer Herstellung, sowie ihre Verwendung als Isocyanatkomponente in Polyurethan-Lacken

Gegenstand der vorliegenden Erfindung sind Isocyanato-isocyanurate der Formel

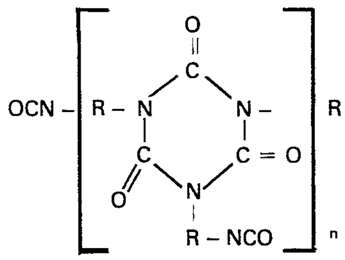

in welcher die einzelnen Reste R gleich oder verschieden sind und Kohlenwasserstoffreste der Formeln

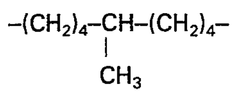

oder

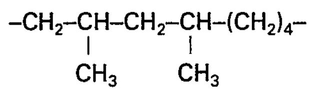

bedeuten und n eine ganze oder gebrochene Zahl von 1 bis 5 bedeutet.

Gegenstand der vorliegenden Erfindung ist auch ein Verfahren zur Herstellung dieser neuen Verbindungen durch Trimerisierung eines Teils der Isocyanat-Gruppen von aliphatischen Diisocyanaten, dadurch gekennzeichnet, dass man als aliphatische Diisocyanate ein im wesentlichen aus einem Diisocyanat der Formel

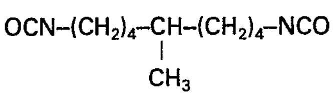

und gegebenenfalls einem Diisocyanat der Formel

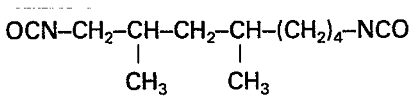

bestehendes Diisocyanat bzw. Diisocyanat-Gemisch verwendet.

Gegenstand der vorliegenden Erfindung ist schliesslich auch die Verwendung der neuen Verbindungen, gegebenenfalls in mit Blockierungsmitteln für Isocyanat-Gruppen blockierter Form, als Isocyanatkomponente in Polyurethan-Lacken.

Isocyanurat-Gruppen aufweisende organische Polyisocyanate gehören zum bekannten Stand der Technik (vgl. z.B. GB-PSen 809 809, 821 158, 837 120, 856 372, 927 173, 944 309, 949 253, 952 931, 954 095, 962 689, 1 391 066, 1 386 399; DE-PS 1 201 992; US-PS 2 801 244; US-PS 3 394 111 oder DE-OS 2 325 826). Ihre Herstellung erfolgt, wie in den beispielhaft genannten Literaturstellen angegeben, im allgemeinen durch katalytische Trimerisierung eines Teils der Isocyanat-Gruppen von organischen Diisocyanaten. Insbesondere die entsprechenden Polyisocyanate mit aliphatisch, bzw. cycloaliphatisch gebundenen Isocyanatgruppen auf Basis handelsüblicher aliphatischer bzw. cycloaliphatischer Diisocyanate wie Hexamethylendiisocyanat oder 3-Isocyanatomethyl-3,5,5-trimethylcyclohexylisocyanat stellen wertvolle Ausgangsmaterialien zur Herstellung von lichtechten Polyurethanlacken dar. Andere in den genannten Vorveröffentlichungen genannte, Isocyanuratgruppen aufweisende Polyisocyanate fanden jedoch für dieses Einsatzgebiet bislang keinen Eingang in die Praxis, da die ihnen zugrundeliegenden Diisocyanate grosstechnisch nicht zur Verfügung stehen oder weit schlechter als die letztgenannten Diisocyanate in praktisch farblose, monomerenfreie, Isocyanuratgruppen aufweisende Polyisocyanate überführt werden können. Dies gilt besonders auch für das in der DE-OS 2 325 826 genannte Isomerengemisch des 2,2,4- und des 2,4,4-Trimethylhexamethylendiisocyanats.

Dieses Isomerengemisch stellt im Unterschied zu den beiden letztgenannten Diisocyanaten kein handelsübliches Diisocyanat dar, da es auf einem umständlichen Weg zugänglich ist und grosstechnisch nicht in der, bei hochwertigen Diisocyanaten üblichen, Reinheit hergestellt werden kann. Bei der technischen Herstellung dieses Isomerengemisches kann die Anwesenheit von chloridhaltigen Verunreinigungen nicht ausgeschlossen werden. Auf diese Verunreinigungen oder auch auf die spezielle Struktur der Diisocyanate ist aller Wahrscheinlichkeit nach der Umstand zurückzuführen, dass bei der Trimerisierung des Isomerengemischs zwecks Herstellung von Isocyanuratgruppen aufweisenden Polyisocyanaten unreine, d.h. stark verfärbte Produkte erhalten werden, die zur Herstellung von hochwertigen Lacken ungeeignet sind.

Auch die in den genannten Literaturstellen beschriebenen Isocyanuratgruppen aufweisenden Polyisocyanate auf Basis von Hexamethylendiisocyanat oder auf Basis von 3-Isocyanatomethyl-3,5,5-trimethylcyclohexylisocyanat weisen jedoch noch erhebliche Nachteile auf, die ihre praktische Einsatzmöglichkeit stark einschränken. So stellt beispielsweise das Trimerisat von IPDI einen hochschmelzenden Festkörper dar, der beispielsweise in flüssigen, lösungsmittelfreien Lacken nicht eingesetzt werden kann. Die bei Raumtemperatur flüssigen Isocyanato-isocyanurate auf Basis von Hexamethylendiisocyanat weisen im allgemeinen eine beschränkte Verträglichkeit mit den in Polyurethanlacken im allgemeinen eingesetzten Polyhydroxylverbindungen auf und sind insbesondere in den oft zur Anwendung gelangenden unpolaren Lösungsmitteln, wie beispielsweise Testbenzin oder Aromaten, nicht oder nur beschränkt löslich.

Es war daher die Aufgabe der vorliegenden Erfindung, neue Polyisocyanate-isocyanurate zur Verfügung zu stellen, die die bekannten Vorteile dieser Verbindungsklasse, wie hohe NCO-Funktionalität, niedriger Dampfdruck, und – im Falle der aliphatischen, bzw. cycloaliphatischen Verbindungen – guten Lichtechtheit, in sich vereinigen, ohne mit den genannten Nachteilen der Verbindungen des Standes der Technik behaftet zu sein.

Diese Aufgabe konnte mit der Bereitstellung der eingangs erwähnten neuen Isocyanato-isocyanurate, bzw. mit dem erfindungsgemässen Verfahren zu ihrer Herstellung gelöst werden.

Ausgangsmaterialien für das erfindungsgemässe Verfahren sind Diisocyanat-Gemische, die im wesentlichen aus einem Diisocyanat der Formel

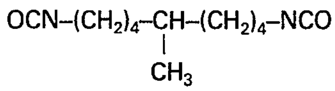

und einem Diisocyanat der Formel

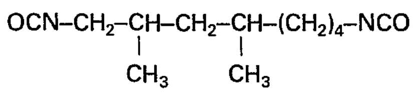

bestehen. Im allgemeinen weisen die beim erfindungsgemässen Verfahren eingesetzten Diisocyanat-Gemische

55–95 Gew.-% 5-Methyl-1,9-diisocyanato-nonan (MNDI),
0–45 Gew.-% 2,4-Dimethyl-1,8-diisocyanato-octan (TMODI) und
0–5 Gew.-% isomere oder homologe Verbindungen dieser Diisocyanate auf.

Reine Diisocyanate der erstgenannten Formel können anstelle der Gemische ebenfalls eingesetzt werden.

Bevorzugtes Ausgangsmaterial ist ein entsprechendes Diisocyanat-Gemisch aus ca. 55 bis 95 Gew.-% 5-Methyl-1,9-diisocyanato-nonan, 5 bis 45 Gew.-% 2,4-Dimethyl-1,8-diisocyanato-octan und 0 bis 5 Gew.-% an isomeren bzw. homologen Diisocyanaten.

Ein beispielsweise gut geeignetes Isomerengemisch aus 90 Gew.-% 5-Methyl-1,9-diisocyanatononan, 9 Gew.-% 2,4-Dimethyl-1,8-diisocyanatooctan und weniger als 1 Gew.-% isomerer bzw. homologer Verbindungen weist einen Siedepunkt bei 0,7 Torr von 115–118 °C auf.

Die Herstellung der Diisocyanat-Gemische erfolgt in an sich bekannter Weise durch Phosgenierung der entsprechenden Diamine. Diese Diamine können ihrerseits durch Umsetzung von 1 Mol Isobutylen mit 2 Mol Acrylnitril und anschliessende Hydrierung des so erhältlichen, olefinisch ungesättigten Bis-nitrils gemäss US-PSen 3880 928, 3880 929, 3896 173 bzw. 3896 174 erhalten werden.

Als Katalysatoren kommen für das erfindungsgemässe Verfahren im Prinzip alle an sich bekannten Trimerisierungskatalysatoren in Betracht. Als Beispiele für derartige Trimerisierungskatalysatoren seien genannt Phosphine, wie in der DE-OS 1 934 763 beschrieben, Alkali- oder Bleisalze gemäss GB-PS 809 809, Alkaliphenolate (GB-Psen 1 391 066 und 1 386 399), Kombinationen aus Alkylenoxid und N,N'-Endoäthylenpiperazin (DE-OS 2 644 684 und US-PS 3 211 703), Aziridin(-derivate) in Kombination mit einem tert. Amin (DE-OS 2 825 826), Mannichbasen, beispielsweise auf Basis von i-Nonylphenol, Formaldehyd und Dimethylamin.

Sehr gut geeignet erwies sich die Verwendung quaternärer Ammoniumhydroxide der allgemeinen Formel

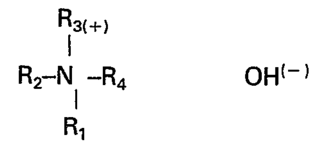

in welcher

$R_1$ für einen Alkylrest mit 1 bis 20, vorzugsweise 4 bis 12 Kohlenstoffatomen, einen araliphatischen Kohlenwasserstoffrest mit 7 bis 10, vorzugsweise 7 Kohlenstoffatomen oder einen gesättigten cycloaliphatischen Kohlenwasserstoffrest mit 4 bis 10, vorzugsweise 5 bis 6 Kohlenstoffatomen steht, von denen jeder mit Hydroxy- und/oder Hydroxyalkylgruppen substituiert sein kann;

$R_2$, $R_3$ und $R_4$ für gleiche oder verschiedene Reste stehen und ggf. Hydroxyl-substituierte Alkylreste mit 1 bis 20, vorzugsweise 1 bis 4, Kohlenstoffatomen bedeuten, wobei zwei der genannten Reste $R_2$, $R_3$ oder $R_4$ auch zusammen mit dem Stickstoffatom und gegebenenfalls zusammen mit einem Sauerstoff- oder einem weiteren Stickstoff-Heteroatom einen heterocyclischen Ring mit 3 bis 5 Kohlenstoffatomen bilden können, oder wobei die Reste $R_2$, $R_3$ und $R_4$ jeweils für Äthylenreste stehen, die zusammen mit dem quartären Stickstoffatom und einem weiteren tertiären Stickstoffatom ein bicyclisches Triäthylen-Diamin-Gerüst bilden können.

Bevorzugte quarternäre Ammoniumhydroxide sind solche der oben genannten Formel, wobei die Reste $R_1$, $R_2$, $R_3$ und $R_4$ die bereits genannte Bedeutung haben, jedoch mit der Massgabe, dass mindestens einer der genannten Reste mindestens eine aliphatisch gebundene Hydroxylgruppe aufweist, die vorzugsweise in 2-Stellung zum quarternären Stickstoffatom angeordnet ist, wobei der Hydroxylsubstituierte Rest, bzw. die Hydroxyl-substituierten Reste ausser den Hydroxyl-Substituenten auch beliebige andere Substituenten, insbesondere $C_1$–$C_4$ Alkoxy-Substituenten, aufweisen können.

Besonders bevorzugte quaternäre Ammoniumhydroxide sind solche der obengenannten Formel, in welchen die Reste $R_1$, $R_2$ und $R_3$ jeweils für Alkylreste der genannten Art und $R_4$ einen Hydroxyäthyl, Hydroxypropyl- oder Hydroxybutylrest bedeuten, in welchem die Hydroxylgruppe vorzugsweise in 2-Stellung zum quaternären Stickstoff angeordnet ist.

Beispiele geeigneter quaternärer Ammoniumhydroxide sind Tetramethylammoniumhydroxid, Tetraäthylenammoniumhydroxid, Trimethylbenzylammoniumhydroxid, Trimethyl-stearylammoniumhydroxid, Dimethyl-äthyl-cyclohexyl-ammoniumhydroxid, N,N,N-Trimethyl-N-(2-hydroxyäthyl)-ammonium-hydroxid, N-Methyl-2-hydroxyäthyl, morpholiniumhydroxid, N-Methyl-N-(2-hydroxypropyl)-pyrrolidiniumhydroxid, N,N,N-Trimethyl-N-(2-hydroxybutyl)-ammoniumhydroxid, N-Dodecyl-tris-(2-hydroxyäthyl)-ammoniumhydroxid, Tetra-(2-hydroxyäthyl)-ammoniumhydroxid, die Verbindung der Formel

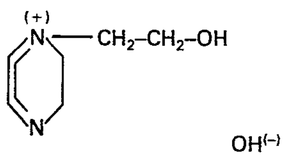

die das Monoaddukt von Äthylenoxid und Wasser an 1,4-Diazabicyclo-[2,2,2]-octan darstellt, sowie ganz besonders bevorzugt N,N-Dimethyl-N-dodecyl-N-(2-hydroxyäthyl)-ammoniumhydroxid und N-(2-Hydroxyäthyl)-N,N-dimethyl-N-(2,2'-dihydroxymethylbutyl)-ammoniumhydroxid.

Die bevorzugt einzusetzenden quartären Ammoniumhydroxide gestatten eine lösungsmittelfreie Trimerisierung der erfindungsgemäss verwendeten aliphatischen Diisocyanate, die in guter Ausbeute unter leicht kontrollierbaren Reaktionsbedingungen (keine Inkubationszeit) zu hellen Produkten führt. Zudem sind die verwendeten Katalysatoren thermolabil, so dass bei Überschreitung gewisser Grenztemperaturen eine selbständige Desaktivierung eintritt. Dadurch kann die Trimerisierungsreaktion ohne oder mit nur geringen Mengen eines Abstoppers nach dem Stand der Technik beendet werden, was zur Folge hat, dass im Verfahrensprodukt keine Trübungen auftreten.

Die Trimerisierungskatalysatoren werden im allgemeinen in Mengen von 0,001–5, vorzugsweise 0,01–2 Gew.-%, bezogen auf Ausgangsdiisocyanat eingesetzt. Bei Verwendung von quaternären Ammoniumhydroxiden als Katalysatoren werden diese im allgemeinen in einer Menge von 0,01–2 Gew.-%, vorzugsweise 0,03–1 Gew.-%, bezogen auf eingesetztes Isocyanat angewendet. Die Mitverwendung von Cokatalysatoren wie Mannichbasen oder Carbamidsäurederivaten ist hierbei möglich.

Die letztgenannten Katalysatoren, bzw. Cokatalysatoren werden vorzugsweise in in geeigneten Lösungsmitteln gelöster Form eingesetzt. Als Lösungsmittel eignen sich je nach Art des Katalysators neben Alkoholen, die bei Eintragen der Katalysatorlösung in das zu trimerisierende Isocyanat zu den bereits erwähnten Carbamidsäurederivaten abreagieren können, beispielsweise Toluol, Dimethylformamid, Dimethylsulfoxid oder auch Mischungen dieser Lösungsmittel. Die keinen reaktionsfähigen Wasserstoff enthaltenden Lösungsmittel dienen im allgemeinen lediglich zum Lösen der Katalysatoren. Sie werden im allgemeinen in Mengen von maximal 5 Gew.-%, bezogen auf eingesetztes Isocyanat, eingesetzt. In erster Näherung handelt es sich somit beim erfindungsgemässen Verfahren im allgemeinen um ein «lösungsmittelfreies» Verfahren. Die ggf. mitverwendeten geringen Mengen an Lösungsmittel werden im Anschluss an die erfindungsgemässe Umsetzung zusammen mit dem überschüssigen Diisocyanat destillativ entfernt, soweit sie nicht in das Verfahrensprodukt eingebaut sind. Die Mitverwendung grösserer Mengen an Lösungsmitteln bei der erfindungsgemässen Umsetzung, die im Anschluss an die Umsetzung destillativ entfernt werden, ist wenig zweckmässig, jedoch andererseits auch nicht völlig ausgeschlossen.

Das erfindungsgemässe Verfahren wird bei Temperaturen von 10 bis 200 °C, vorzugsweise von 40 bis 160 °C durchgeführt.

Im folgenden wird das erfindungsgemässe Verfahren beispielhaft beschrieben:

Das Ausgangsdiisocyanat wird unter Inertgas (dessen Mitverwendung nicht unbedingt erforderlich ist) vorgelegt, und auf eine Temperatur im Bereich von 10–100 °C, beispielsweise 50 °C, gebracht. In die Reaktionslösung trägt man die bevorzugt einzusetzende Lösung des quaternären Ammoniumhydroxid-Katalysators ein, worauf augenblicklich die Trimerisierung beginnt. Die Temperatur steigt dabei auf 60–120 °C an. Je nach Katalysatormenge und/oder Reaktionstemperatur erreicht der Ansatz in 0,5 bis 5 Stunden den gewünschten NCO-Wert. Die Trimerisierungsreaktion bricht dann durch thermische Desaktivierung des Katalysators von selbst ab oder kann durch Zugabe eines Abstoppers wie z.B. Säuren wie Perfluorbutansulfonsäure besser aber kurzzeitiges Erhitzen auf Temperaturen von 80–180 °C unterbrochen werden. Danach wird vorzugsweise die Trimerisatlösung im Hochvakuum von überschüssigen Monomeren befreit, und man erhält als Destillationsrückstand die erfindungsgemässen Trimerisate.

Ausschlaggebend für die Qualität des Endproduktes (Viskosität und Farbe) ist der schonende Verlauf der Trimerisierung. Die Katalysatormenge wird deshalb so dosiert, dass der gewünschte NCO-Wert nicht zu schnell erreicht wird. Zweckmässig ist es darum, die Trimerisierung – wie oben beschrieben – nur mit einem Teil der Katalysatormenge zu initiieren, nach Erreichen eines ersten Temperaturmaximums bei dieser Temperatur nachzurühren bis die Reaktion abklingt, um dann weitere Katalysatorlösung, mit dem Erfolg erneuter Temperaturzunahme, zuzugeben. Ist der gewünschte NCO-Gehalt der Trimerisat-Lösung zu diesem Zeitpunkt noch nicht erreicht, lässt sich nach der gleichen Methode, bei Verwendung von thermolabilen Katalysatoren bevorzugt im Bereich von deren spezifischer Zersetzungstemperatur, der Endpunkt einstellen. Nach thermischem Abstoppen oder Zugabe eines an sich bekannten Abstoppers kann das reine Trimerisat durch an sich bekannte Methoden, wie etwa Dünnschichtdestillation, als praktisch farbloses, mittel- bis niedrigviskoses Harz in Form des Destillationsrückstandes erhalten werden.

Im allgemeinen wird die Trimerisierungsreaktion bis zu einem NCO-Gehalt des Reaktionsgemisches von 20 bis 30 Gew.-% geführt. Wie bereits angedeutet, erfolgt vorzugsweise anschliessend eine destillative Entfernung des dann noch im Gemisch vorliegenden Ausgangsdiisocyanats.

IR-Spektren der erfindungsgemässen Produkte beweisen deren Isocyanuratstruktur durch Absorptionen bei 1687 $cm^{-1}$ für die C=O-Schwingung bei 1456 $cm^{-1}$ für die «Ringbande» des gebildeten Sechsrings. Gleichzeitig kann die Bildung wesentlicher Mengen an dimeren Produkten (Uretdion) ausgeschlossen werden.

Die genaue Zusammensetzung des gebildeten Produktgemisches geht aus gelchromatographischen Untersuchungen hervor, wonach im allgemeinen einen Isocyanuratring aufweisende Triisocyanate (n = 1) die Hauptkomponente darstellen. Nach destillativer Entfernung des nicht umgesetzten Diisocyanats liegen neben dieser Hauptkomponente, je nach Trimerisierungsgrad, unterschiedliche Mengen an mehr als einen Isocyanuratring aufweisenden Polyisocyanaten (n = 1–5) vor. In den bevorzugten, erfindungsgemässen Isocyanato-isocyanuraten liegt der Gehalt an freiem Ausgangsdiisocyanat unter 5 Gew.-%, vorzugsweise unter 2 Gew.-%, während n im statistischen Mittel einen Wert von 1,2 bis 3 aufweist. Der NCO-Gehalt der erfindungsgemässen Produkte liegt im allgemeinen zwischen 10 und 16 Gewichtsprozent.

Die erfindungsgemässen Isocyanat-isocyanurate besitzen eine Reihe vorteilhafter Eigenschaften, die sie als Isocyanathärter für Beschichtungssysteme nach dem Isocyanat-Polyadditionsverfahren hervorragend geeignet machen. Auf Grund ihrer geringen Viskosität, die in Abhängigkeit vom Trimerisierungsgrad schwankt, jedoch im allgemeinen unter 10,000 mPas (25 °C) liegt, werden für ihren Einsatz keine oder nur geringe Mengen an üblichen Lösungsmitteln benötigt. Systeme mit den erfindungsgemässen Produkten sind daher umweltfreundlich und kostengünstig.

Einer Anwendung der erfindungsgemässen Polymerisate für Beschichtungssysteme kommt weiterhin zugute, dass es sich um klare und farblose, bzw. nur schwach gefärbte Produkte handelt, die etwa in Zweikomponenten-Polyurethanlacken hoher Qualität eingesetzt werden können. Lacke mit einem Gehalt an erfindungsgemässen Trimerisaten zeigen neben rascher chemischer Trocknung und sehr guter mechanischer Stabilität eine hohe Witterungsbeständigkeit sowie ganz besonders eine hohe UV-Stabilität.

Der wesentliche Vorteil gegenüber Polyisocyanaten nach dem Stand der Technik liegt jedoch in der besseren Verträglichkeit der Produkte mit unpolaren Lösungsmitteln, die die Anwendungsbreite dieser gegenüber herkömmlichen Produkten wesentlich vergrössert, da auf den Einsatz teurer, evtl. physiologisch bedenklicher Lösungsmittel verzichtet werden kann.

Der folgende Vergleich der erfindungsgemässen Trimerisate, kurz als MNDI-Trimerisat bezeichnet, mit unter gleichen Bedingungen hergestelltem Hexamethylendiisocyanat (HDI)-Trimerisat zeigt deutlich die bessere Verträglichkeit der erfindungsgemässen Trimerisate gegenüber unpolaren Lösungsmitteln. In Parallelversuchen wurden je 30 g Polymerisat, teilweise gelöst in Äthylenglykolacetat (ÄGA), vorgelegt und unter Rühren langsam mit n-Hexan, bzw. Lackbenzin (ein der Lösungsmittelverordnung vom 26.2.1954 entsprechendes Testbenzin, das nicht als gesundheitsschädlich einzuordnen ist) versetzt, bis gerade eben eine bleibende, auch nach längerem Rühren beständige, Trübung auftrat. Die nachfolgende Tabelle zeigt die unter diesen Versuchsbedingungen benötigten Mengen an Verdünner in Millilitern:

| | n-Hexan | Lackbenzin |
|---|---|---|
| HDI-Trim. | 7,15 | 7,1 |
| MNDI-Trim. | 22,5 | 42,0 |
| HDI-Trim. (80% in ÄGA) | 12,9 | |
| MNDI-Trim. (80% in ÄGA) | 42,0 | |

Die erfindungsgemässen Polyisocyanate stellen wertvolle Ausgangsmaterialien zur Herstellung von Polyurethankunststoffen nach dem Isocyanat-Polyadditionsverfahren, insbesondere zur Herstellung von Ein- oder Zweikomponenten-Polyurethanlacken, sowie in mit an sich bekannten Blokkierungsmitteln blockierter Form wertvolle Ausgangsmaterialien für Zweikomponenten-Polyurethan-Einbrennlacke dar.

Bevorzugte Reaktionspartner für die erfindungsgemässen, gegebenenfalls in blockierter Form vorliegenden Verfahrensprodukte bei der Herstellung von Polyurethanlacken sind die in der Polyurethanlacktechnik an sich bekannten Polyhydroxypolyester und -äther, Polyhydroxypolyacrylate und gegebenenfalls niedermolekularen, mehrwertigen Alkohole. Auch Polyamine, insbesondere in blockierter Form als Polyketimine oder Oxazolidine sind denkbare Reaktionspartner für die erfindungsgemässen Verfahrensprodukte. Die Mengenverhältnisse, in welchen die erfindungsgemässen, gegebenenfalls blockierten Polyisocyanate und die genannten Reaktionspartner bei der Herstellung von Polyurethanlacken umgesetzt werden, werden im allgemeinen so gewählt, dass auf eine (gegebenenfalls blockierte) Isocyanatgruppe 0,8–3, vorzugsweise 0,9–1,1, Hydroxy-, Amino-, und/oder Carboxygruppen entfallen.

Zur Beschleunigung der Aushärtung können in bekannter Weise die in der Isocyanatchemie üblichen Katalysatoren verwendet werden, wie z.B. tert. Amine wie Triäthylamin, Pyridin, Methylpyridin, Benzyldimethylamin, N,N-Dimethylaminocyclohexan, N-Methylpiperidin, Pentamethyldiäthylentriamin, N,N'-Endoäthylenpiperazin, N,N'-Dimethylpiperazin usw., Metallsalze wie Eisen(III)-chlorid, Zinkchlorid, Zink-2-äthylcaproat, Zinn(II)-2-äthylcaproat, Dibutylzinn(IV)-dilaurat, Molybdänglykolat, usw.

Bei Verwendung der erfindungsgemässen Verfahrensprodukte in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z.B. 40 bis 140 °C), gegebenenfalls in Gegenwart eines geeigneten Katalysators, wie z.B. tert.-Amine, Metallsalze wie Zink-2-äthylcaproat, Zinn(II)-2-äthyl-caproat, Dibutylzinn (IV)-dilaurat oder Alkaliphenolat umgesetzt.

Geeignete Blockierungsmittel sind beispielsweise:

Monophenole wie Phenol, die Kresole, die Trimethylphenole, die tert. Butylphenole; tertiäre Alkohole wie tert. Butanol, tert. Amylalkohol, Dimethylphenylcarbonol; leicht Enole bildende Verbindungen wie Acetessigester, Acetylaceton, Malonsäurederivate wie Malonsäurediester mit 1 bis 8 C-Atomen in den Alkoholresten; sekundäre aromatische Amine wie N-Methylanilin, die N-Methyltoluidine, N-Phenyltoluidin, N-Phenylxylidin; Imide wie Succinimid; Lactame wie ε-Caprolactam, δ-Valerolactam; Oxime wie Butanonoxim, Cyclohexanonoxim; Mercaptane wie Methylmercaptan, Äthylmercaptan, Butylmercaptan, 2-Mercaptobenzthiazol, α-Naphthylmercaptan, Dodecylmercaptan, Triazole wie 1H-1,2,4-Triazol.

Zur Herstellung der Lackbindemittel werden gegebenenfalls blockiertes Polyisocyanat, polyfunktioneller Reaktionspartner, Katalysator und gegebenenfalls die üblichen Zusätze, wie z.B. Pigmente, Farbstoffe, Füllstoffe und Verlaufmittel miteinander auf einem üblichen Mischaggregat, wie z.B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z.B. Streichen, Rollen, Giessen, Spritzen, dem Wirbelsinterverfahren oder dem elektrostatischen Pulversprühverfahren auf den zu beschichtenden Gegenstand aufgebracht werden.

Die die erfindungsgemässen Polyisocyanate enthaltenden Lacke ergeben Filme, die überraschend gut auf metallischem Untergrund haften, besonders lichtecht, wärmefarbstabil und sehr abriebfest sind. Darüber hinaus zeichen sie sich durch grosse Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffend Gewichtsprozente.

In den Beispielen werden folgende Katalysatorlösungen eingesetzt:

A 2-Hydroxyäthyl-dodecyldimethylammoniumhydroxid, durch Äthoxylierung von n-Dodecyldimethylamin («Domin») in $H_2O/CH_3OH$ bei ca. 40 °C hergestellt, verdünnt auf eine ca. 2 Gew.-% Lösung mit 2-Äthylhexanol/Methanol (8:1)

B wie A, jedoch verdünnt auf eine ca. 5 Gew.-% Lösung

C wie A, jedoch Dimethylformamid/Methanol (8:1) als Lösungsmittel

D N,N-Dimethyl-N-(2,2-dimethylol-butyl)-N-(2-hydroxyäthyl)-ammoniumhydroxid, dargestellt durch Äthoxylierung von N,N-Dimethyl-N-(2,2-dimethylolbutyl)-amin, als ca. 6 Gew.-% Lösung in 2-Äthylhexanol/Methanol (8:1).

Bezüglich der Diisocyanate wurden folgende Abkürzungen verwendet:

MNDI: 5-Methyl-1,9-diisocyanato-nonan;

DMODI: 2,4-Dimethyl-1,8-diisocyanato-octan.

Beispiel 1

22,4 g (0,1 Mol) eines Isocyanatgemisches bestehend aus ca. 60% MNDI und ca. 40% DMODI (+Spuren von Trimethylheptyldiisocyanat-Isomeren) werden bei 50 °C mit 3 ml Katalysatorlösung A versetzt, wobei die Temperatur des Gemisches innerhalb von 10 Minuten auf 65 °C ansteigt. Nach weiteren 45 Min. bei dieser Temperatur ist der NCO-Gehalt der Lösung auf 26,5 % gesunken. Nach 20 Minuten Erhitzen auf 90 °C hat sich ein NCO-Wert von 25,9% eingestellt, der sich auch bei weiterem Erwärmen nicht mehr verändert.

Nach Entfernung überschüssiger Monomerer erhält man das Polyisocyanat in Form einer niedrigviskosen, klaren Flüssigkeit mit schwachgelber Eigenfärbung (NCO-Gehalt 10,8%).

Beispiel 2

100 g (0,45 Mol des Isocyanatgemisches aus Beispiel 1 wird bei 65 °C mit 9 ml Katalysatorlösung A versetzt. Nach 10 Minuten ist die Temperatur auf 70 °C gestiegen, der NCO-Wert des Gemisches beträgt nach weiteren 20 Min., in denen die Temperatur langsam auf 64 °C sinkt, 31,4%. Bei 64 °C werden weitere 3 ml Katalysatorlösung zugegeben, wobei unter erneuter exothermer Reaktion der NCO-Wert auf 28,2% sinkt. Nach Entfernung monomerer Isocyanate beträgt der NCO-Wert des klaren, praktisch farblosen Rückstandes 11,5%.

Beispiel 3

100 g (0,45 Mol) des Isocyanatgemisches aus Beispiel 1 werden bei 50 °C mit 10 ml Katalysatorlösung B vernetzt, worauf die Temperatur auf 60 °C steigt. Der NCO-Wert beträgt nach 30 Minuten 29,5%. Zugabe von weiteren 2 ml Katalysatorlösung bewirkt einen Temperaturanstieg auf 65 °C und eine Abnahme des NCO-Wertes auf 26,4% nach weiteren 20 Min. Im Verlauf von 40 Min. wird die Temperatur der Lösung auf 100 °C erhöht, worauf sich ein NCO-Wert von 20,6% einstellt, der sich nach weiteren 15 Min. bei 90 °C nur unwesentlich verändert. Der NCO-Wert des von Monomeren weitgehend befreiten Produkts beträgt 12,2%.

Beispiel 4:

22,4 (0,1 Mol) des in Beispiel 1 beschriebenen Diisocyanatgemisches werden 50% in Äthylenglykolacetat gelöst und bei 67° C mit 2,5 ml Katalysatorlösung B versetzt. Die Temperatur der Lösung steigt auf 73 ° C, der NCO-Wert der Lösung beträgt

nach 45 Min. 11,3%. Zur Desaktivierung des Katalysators wird die Lösung 15 Min. auf 100 °C erhitzt. Nach anschliessender Entfernung von Lösungsmittel sowie Monomeren erhält man niedrigviskoses, schwach gelbes Harz mit einem NCO-Wert von 13,4%.

Beispiel 5

22,4 g (0,1 Mol) Isocyanat-Isomerengemisch wie in Beispiel 1 werden mit zwei Tropfen einer Mannich-Base aus i-Nonyl-phenol, Formaldehyd und Dimethylamin versetzt. Bei 60 °C werden 1,5 ml Katalysatorlösung A zugegeben. Die Temperatur des Gemisches steigt auf 64 °C. Nach Abklingen der exothermen Reaktion wird die Temperatur durch Heizen auf 65 °C gehalten. Bei dieser Temperatur wird erneut 1 ml Katalysatorlösung zugegeben.

Nach 1 Stunde beträgt der NCO-Gehalt des Gemisches 27,1%. Das von Monomeren weitgehend befreite Produkt von schwachgelber Eigenfärbung besitzt einen NCO-Gehalt von 14,6%.

Beispiel 6

168,5 g (ca. 0,75 Mol) eines Isomerengemisches aus ca. 94% MNDI, ca. 4% DMODI und ca. 2% Trimethyldiisocyanatoheptan-Isomeren wird bei 50 °C vorgelegt. Nach Zugabe von 12,5 ml Katalysatorlösung B steigt die Temperatur auf 60 °C. Nachkatalyse mit einmal 5 ml und einmal 10 ml Katalysatorlösung ergibt bei einer Gesamtreaktionszeit von 100 Min. einen NCO-Wert des Gemisches von 26,9%. Durch Zugabe von 0,2 ml einer Abstopperlösung (bestehend aus 1 ml $C_4F_9SO_3H$ in 2 ml DMF) wird die Reaktion unterbrochen. Der NCO-Gehalt des durch Dünnschichtdestillation von Monomeren befreiten Produktes (0,7% Restgehalt) beträgt 11,0%.

Beispiel 7

1120 g (5 Mol) Isocyanatgemisch bestehend aus ca. 88% MNDI, 10% DMODI und 2% weiteren OCN $-(-C_{10}H_{20}-)-$NCO-Isomeren werden bei 90 °C portionsweise mit insgesamt 90 ml Katalysatorlösung D versetzt. Bei Temperaturen zwischen 90 und 115 °C sinkt der NCO-Wert innerhalb einer Stunde auf 25,5%. Die Trimerisierungsreaktion wird durch Zugabe von 0,5 ml Abstopperlösung (bestehend aus 1 ml $C_4F_9SO_3H$ in 2 ml DMF) unterbrochen.

Beispiel 8

896 g (4 Mol) Isomerengemisch aus Beispiel 7 wird wie dort mit insgesamt 70 ml Katalysatorlösung bis zu einem NCO-Gehalt von 27,6% trimerisiert (0,3 ml Abstopperlösung). Die Trimerisatlösungen aus Beispiel 7 und Beispiel 8 wurden gemeinsam einer Dünnschichtdestillation unterworfen. Man erhält ein praktisch farbloses Harz mit einem NCO-Gehalt von 12,3% und einer Viskosität von 5040 mPas bei 25 °C (Restmonomerengehalt: 0,55%).

Beispiel 9

224 g (1 Mol) Diisocyanat-Isomerengemisch aus Beispiel 7 wird mit 0,5 ml einer Mannich-Base, dargestellt aus i-Nonylphenol, Formaldehyd und Dimethylamin versetzt und auf 70 °C erwärmt. Nach Zugabe von 10 ml Katalysatorlösung C steigt die Temperatur auf 88 °C, um dann langsam wieder abzufallen. Nach Zugabe weiterer 3 ml Katalysatorlösung ist nach insgesamt 45 Min. Reaktionszeit der NCO-Wert des Gemisches auf 27,4% gesunken. Die Trimerisierung wird durch Zugabe von 0,1 ml Säurelösung (1 ml $C_4F_9SO_3H$ in 2 ml 2-Äthylhexanol) abgestoppt. Der NCO-Gehalt des durch Dünnschichtdestillation von Monomeren bis auf einen Monomerengehalt von 0,6% befreiten Produkts beträgt 13,2%.

Beispiel für die Darstellung der erfindungsgemäss verwendeten Diisocyanate aus den entsprechenden Diaminen.

Beispiel 10

In eine Lösung von 57,5 g (0,33 Mol) Diamin, bestehend aus ca. 90% 5-Methyl-1,10-diaminononan und ca. 10% 2,4-Dimethyl-1,8-diamino-octan, in 1151 g wasserfreiem o-Dichlorbenzol wird bei 80–85 °C ein mässig starker $CO_2$-Strom eingeleitet, bis kein $CO_2$ mehr aufgenommen wird. (Ca. 90 Min., Ausfällung des Carbamidsäuresalzes). Unter Einleiten eines Phosgenstromes wird eine Stunde bei Raumtemperatur, dann unter Rückfluss bis zum Entstehen einer klaren Lösung (ca. 4,5 h) gerührt. Nach Ausblasen mit Stickstoff und Abziehen des o-Dichlorbenzols im Wasserstrahlvakuum wird der Rückstand im Hochvakuum destilliert. ($Kp_{0,2}$: 105–106 °C). Man erhält 68 g Diisocanat(-gemisch) entsprechend einer Ausbeute von 91% in Form einer farblosen, klaren Flüssigkeit.

**Patentansprüche**

1. Isocyanato-isocyanurate der Formel

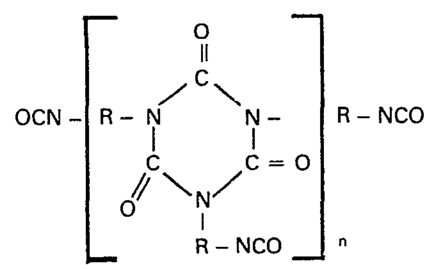

in welcher die einzelnen Reste R gleich oder verschieden sind und Kohlenwasserstoffreste der Formeln

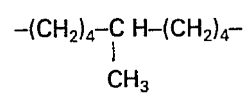

oder

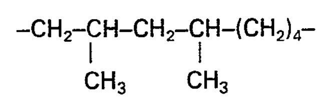

bedeuten und n eine ganze oder gebrochene Zahl von 1 bis 5 bedeutet.

2. Verfahren zur Herstellung von Isocyanato-isocyanuraten gemäss Anspruch 1 durch katalytische Trimerisierung eines Teils der Isocyanat-Gruppen von aliphatischen Diisocyanaten, dadurch gekennzeichnet, dass man als aliphatische Diisocyanate ein im wesentlichen aus einem Diisocyanat der Formel

$$OCN-(CH_2)_4-\underset{\displaystyle CH_3}{\underset{|}{C}}H-(CH_2)_4-NCO$$

und gegebenenfalls einem Diisocyanat der Formel

$$OCN-CH_2-\underset{\displaystyle CH_3}{\underset{|}{C}}H-CH_2-\underset{\displaystyle CH_3}{\underset{|}{C}}H-(CH_2)_4-NCO$$

bestehendes Diisocyanat bzw. Diisocyanat-Gemisch verwendet.

3. Verwendung der Isocyanato-isocyanurate gemäss Anspruch 1, gegebenenfalls in mit Blockierungsmittel für Isocyanat-Gruppen blockierter Form, als Isocyanatkomponente in Polyurethan-Lacken.

**Revendications**

1. Isocyanato-isocyanurates de formule:

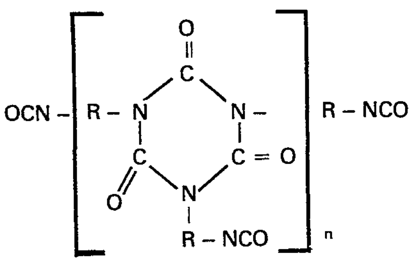

dans laquelle:
les divers restes R sont égaux ou différents et désignent des restes hydrocarbonés de formules:

$$-(CH_2)_4-\underset{\displaystyle CH_3}{\underset{|}{C}}H-(CH_2)_4-$$

ou

$$-CH_2-\underset{\displaystyle CH_3}{\underset{|}{C}}H-CH_2-\underset{\displaystyle CH_3}{\underset{|}{C}}H-(CH_2)_4-$$

et n est un nombre entier ou fractionnaire ayant une valeur de 1 à 5.

2. Procédé de production d'isocyanato-isocyanurates suivant la revendication 1 par trimérisation catalytique d'une partie des groupes isocyanate de diisocyanates aliphatiques, caractérisé en ce qu'on utilise comme diisocyanates aliphatiques un diisocyanate ou un mélange de diisocyanates formé principalement d'un diisocyanate de formule:

$$OCN-(CH_2)_4-\underset{\displaystyle CH_3}{\underset{|}{C}}H-(CH_2)_4-NCO$$

et, le cas échéant, d'un diisocyanate de formule:

$$OCN-CH_2-\underset{\displaystyle CH_3}{\underset{|}{C}}H-CH_2-\underset{\displaystyle CH_3}{\underset{|}{C}}H-(CH_2)_4-NCO$$

3. Utilisation des isocyanato-isocyanurates suivant la revendication 1, éventuellement sous la forme protégée avec un agent de protection pour des groupes isocyanate, comme composant isocyanate dans des peintures à base de polyuréthanne.

**Claims**

1. Isocyanato-isocyanurates corresponding to the formula

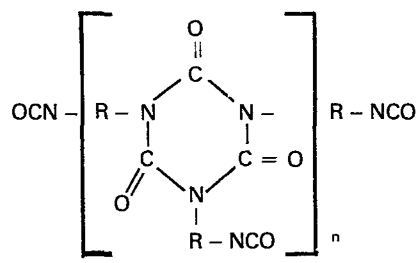

in which the individual radicals R are the same or different and represent hydrocarbon radicals of the formulae

$$-(CH_2)_4-\underset{\displaystyle CH_3}{\underset{|}{C}}H-(CH_2)_4-$$

or

$$-CH_2-\underset{\displaystyle CH_3}{\underset{|}{C}}H-CH_2-\underset{\displaystyle CH_3}{\underset{|}{C}}H-(CH_2)_4-$$

and n is an integer or non-integer of from 1 to 5.

2. A process for producing the isocyanato-isocyanurates claimed in Claim 1 by catalytically trimerising some of the isocyanate groups in aliphatic diisocyanates, characterised in that the aliphatic diisocyanate used is a diisocyanate or diisocyanate mixture consisting essentially of a diisocyanate corresponding to the formula

$$OCN-(CH_2)_4-\underset{\displaystyle CH_3}{\underset{|}{C}}H-(CH_2)_4-NCO$$

and optionally a diisocyanate corresponding to the formula

$$OCN-CH_2-\underset{\displaystyle CH_3}{\underset{|}{C}}H-CH_2-\underset{\displaystyle CH_3}{\underset{|}{C}}H-(CH_2)_4-NCO$$

3. The use of the isocyanato-isocyanurates claimed in Claim 1, which may be blocked with blocking agents for isocyanate groups, as isocyanate component in polyurethane lacquers.